# EUROPÄISCHE PATENTSCHRIFT

(11) **EP 3 361 952 B1**
(45) Veröffentlichungstag und Bekanntmachung des Hinweises auf die Patenterteilung: **08.04.2020**
(21) Anmeldenummer: 15816109.1
(22) Anmeldetag: 09.12.2015
(51) Int. Cl.: A61B 6/03

(54) **TOMOGRAFIEANLAGE UND VERFAHREN ZUM ERZEUGEN EINER ABFOLGE VON VOLUMENBILDERN EINES GEFÄSSSYSTEMS**
TOMOGRAPHY SYSTEM AND METHOD FOR PRODUCING A SERIES OF VOLUME IMAGES OF A VASCULAR SYSTEM
SYSTÈME TOMOGRAPHIQUE ET PROCÉDÉ DE GÉNÉRATION D'UNE SÉQUENCE D'IMAGES EN VOLUME D'UN SYSTÈME VASCULAIRE

(30) Priorität: 03.12.2015 DE 102015224176
(43) Veröffentlichungstag der Anmeldung: 22.08.2018
(73) Patentinhaber: Siemens Healthcare GmbH, 91052 Erlangen (DE)
(72) Erfinder: KLINGENBECK, Klaus, 91347 Aufseß (DE); KOWARSCHIK, Markus, 90408 Nürnberg (DE); ROHKOHL, Christopher, 6364 Brixen im Thale (AT); ROYALTY, Kevin, Fitchburg, WI 53711 (US); SCHAFER, Sebastian, Madison, WI 53711 (US)
(86) Internationale Anmeldenummer: PCT/EP2015/079102
(87) Internationale Veröffentlichungsnummer: WO 2017/092835

(56) Entgegenhaltungen:
- DE-A1-102012 217 613
- DE-A1-102013 226 858
- US-A1- 2005 226 484

## Beschreibung

Die Erfindung betrifft eine Tomografieanlage mit einem ersten und einem zweiten Strahlquelle-Detektor-Paar und einem Volumenbilderzeugungssystem zum Erzeugen einer Abfolge von Volumenbildern eines Gefäßsystems. Das erste Strahlquelle-Detektor-Paar ist zum Erfassen einer Abfolge von ersten Projektionsbilddatensätzen mit ersten Pixelwerten aus einem ersten Projektionswinkel vorbereitet. Das zweite Strahlquelle-Detektor-Paar ist zum Erfassen aus einem zweiten Projektionswinkel einer Abfolge von zweiten Projektionsbilddatensätzen mit zweiten Pixelwerten vorbereitet, wobei sich der zweite Projektionswinkel von dem ersten Projektionswinkel unterscheidet.

Jeder der Projektionsbilddatensätze kann als Datensatz eines (radiografischen) Schattenbilds angesehen werden, auf dem Strukturen, die im Strahlengang hintereinander liegen, Schatten erzeugen, die sich überlagern. Die Aufnahme je eines Projektionsbilddatensatzes aus zwei unterschiedlichen Projektionswinkeln kann (in Analogie zu Quadraturempfangsverfahren) zumindest dann als Inphase-Quadratur-Aufnahmeverfahren (I&Q-Aufnahmeverfahren) angesehen werden, wenn sich die beiden Projektionswinkel um 90° unterscheiden. Unter einer Abfolge von Volumenbildern wird üblicherweise eine Folge von mindestens zwei dreidimensionalen Volumenbildern verstanden, die zeitlich aufeinanderfolgen. Hierbei zeigt ein erstes der mindestens zwei dreidimensionalen Volumenbilder beispielsweise eine Anflutungsphase und ein zweites der mindestens zwei dreidimensionalen Volumenbilder beispielsweise eine Ausflussphase. Die Tomografieanlage kann beispielsweise eine Röntgen-Tomografieanlage oder eine Fluoreszenz-Tomografieanlage sein.

Die Strahlquelle und der Detektor des ersten Strahlquelle-Detektor-Paars können jeweils an einem gemeinsamen beweglichen Träger, beispielsweise einem selben C-Bogen oder an unterschiedlichen beweglichen Trägern, beispielsweise an je einem Roboterarm, befestigt sein. Entsprechendes gilt für die Strahlquelle und den Detektor des zweiten Strahlquelle-Detektor-Paars. Eine Ausführungsform sieht vor, dass beide Strahlquelle-Detektor-Paare an einem gemeinsamen beweglichen Träger, beispielsweise einem selben C-Bogen, befestigt sind. Optional kann an dem ersten und/oder dem zweiten Detektor jeweils ein Bildverstärker angeschlossen sein. Eine davon unabhängige Option sieht vor, dass der erste und/oder der zweite Detektor einen Bildverstärker umfasst.

Außerdem betrifft die Erfindung ein Verfahren zum Erzeugen einer Abfolge von Volumenbildern eines Gefäßsystems, wobei das Verfahren Folgendes umfasst: Erfassen einer Abfolge von ersten Projektionsbilddatensätzen mit ersten Pixelwerten aus einem ersten Projektionswinkel, Erfassen einer Abfolge von zweiten Projektionsbilddatensätzen mit zweiten Pixelwerten aus einem zweiten Projektionswinkel, wobei sich der zweite Projektionswinkel von dem ersten Projektionswinkel unterscheidet, und Erzeugen der Abfolge von Volumenbildern.

In der Diagnostik und Therapie werden immer höhere Anforderungen an die Leistungsfähigkeit medizinischer Geräte gestellt. Damit wird insbesondere das Ziel verfolgt, Gesundheitsgefahren und Personenschäden infolge fehlerhafter Diagnose oder Behandlung zu vermeiden sowie Belastungen von Patienten und behandelnden Personen in der Anwendung von diagnostischen und therapeutischen Verfahren zu minimieren.

Aus der DE 10 2013 226 858 A1 ist ein Verfahren zur Erzeugung eines wenigstens dreidimensionalen Anzeigedatensatzes eines auf die zeitliche Ausbreitung eines Kontrastmittels in einem Gefäßsystem bezogenen Zeitparameters bekannt. Dabei wird eine Serie von die Ausbreitung zeigenden Röntgenbildern der digitalen Subtraktionsangiographie (DSA) aus wenigstens zwei unterschiedlichen Projektionsrichtungen verwendet. Zunächst wird eine Position eines in jeweils wenigstens einem Röntgenbild definierten Korrespondenzpunktes ermittelt. Dann wird ein dieser Position zugeordneter Zeitparameter ermittelt durch Auswertung von den Korrespondenzpunkten zugeordneten Zeit-Intensitätskurven. Schließlich wird der aus den Positionen und den zugeordneten Zeitparametern gebildete Anzeigedatensatz angezeigt.

Aus der US 2005 / 0 226 484 A1 ist ein Verfahren zur Erzeugung einer Varianzkarte aus Projektionsdaten eines Tomografiesystems bekannt. Dazu kann mittels eines statistischen Modells ein auf den Projektionsdaten basierendes Varianzmaß formuliert werden. Weiterhin kann eine durch Rausch- und Artefaktfaktoren verursachte Variabilität eines durchschnittlichen Pixelwertes bestimmt werden. Beim Formulieren des Varianzmaßes können auch verschiedene weitere Quelle eines Rauschens oder eines Unsicherheitsmaßes modelliert und aus gemessenen Projektionsdaten berechnet werden. Ein solches Unsicherheitsmaß kann dabei auf einer Pfadlänge oder einer integrierten Dämpfung basieren.

Aus der DE 10 2012 217 613 A1 ist ein Verfahren zur Reduzierung von Artefakten bei der Rekonstruktion von Bilddatensätzen aus mehreren, unter unterschiedlichen Projektionsrichtungen aufgenommenen Projektionsbildern bekannt. Dabei wird eine über die Projektionsbilddaten legbare Konfidenzmaske ermittelt, welche für jedes Pixel einen Verlässlichkeitswert für das Projektionsdatum des Pixels enthält. Weiterhin werden korrigierte Projektionsdaten als gemäß dem Verlässlichkeitswert gewichtete Summe des ursprünglichen Projektionsdatums und eines durch Auswertung eines Operators ermittelten Korrekturdatums ermittelt.

Die US 2013/0237815 A1 beschreibt ein Verfahren zum Ermitteln eines vierdimensionalen Angiographie-Datensatzes mittels einer Biplan-Röntgeneinrichtung. Hierbei wird mit zwei Aufnahmeanordnungen, die in unterschiedliche Projektionsrichtungen ausgerichtet sind, ein Kontrastmittel in einer Anflutungsphase und/oder Ausflussphase zeitaufgelöst aufgenommen und durch Rückprojektion ein animierter dreidimensionaler Rekonstruktionsdatensatz gewonnen. Es wird vorgeschlagen, Flussinformationen für diejenigen Zeiträume zu extra- oder interpolieren, in denen Gefäßabschnitte nicht sichtbar sind.

Die US 7,519,414 B2 beschreibt ein DSA-System zur angiografischen Darstellung eines Blutgefäßsystems eines Patienten aus mindestens zwei verschiedenen Projektionsrichtungen. Um während einer endovaskulären Intervention mit einem Mikrokatheter die Notwendigkeit einer Applikation von Kontrastmittel über die Katheterspitze zu vermeiden, wird vorgeschlagen, in eine 3D-Darstellung des Blutgefäßsystems eine aus mehreren Röntgenbildern rekonstruierte dreidimensionale Darstellung des Mikrokatheters einzublenden, die in einer angiografischen Voruntersuchung gewonnen wurde. Die zeitliche Entkopplung der angiografischen Voruntersuchung und der endovaskulären Intervention vermeidet eine Applikation von Kontrastmittel während des therapeutischen Eingriffs.

Es ist eine Aufgabe der vorliegenden Erfindung, eine Tomografieanlage bereitzustellen, mit der eine Abfolge von Volumenbildern erzeugt werden kann, deren Volumenbilder eine höhere Abbildungstreue aufweisen als Volumenbilder von Abfolgen von Volumenbildern, die unter gleichen Voraussetzungen mit bekannten Tomografieanlagen erzeugt werden können.

Diese Aufgabe wird erfindungsgemäß durch eine Tomografieanlage gelöst, die ein erstes Strahlquelle-Detektor-Paar zum Erfassen einer Abfolge von ersten Projektionsbilddatensätzen mit ersten Pixelwerten aus einem ersten Projektionswinkel, ein zweites Strahlquelle-Detektor-Paar zum Erfassen aus einem zweiten Projektionswinkel einer Abfolge von zweiten Projektionsbilddatensätzen mit zweiten Pixelwerten und einem Volumenbilderzeugungssystem zum Erzeugen einer Abfolge von Volumenbildern eines Gefäßsystems aufweist, wobei sich die beiden Projektionswinkel unterscheiden. Die Tomografieanlage ist dabei dazu eingerichtet, die Abfolgen der ersten und zweiten Projektionsbilddatensätze während eines synchronen und rotationswinkelversetzten Umlaufs oder Teilumlaufs der beiden Strahlquelle-Detektor-Paare zu erfassen, wobei für beide Strahlquelle-Detektor-Paare gilt, dass der jeweilige Detektor in einer Rotationsebene umläuft, die zu der Rotationsebene beabstandet ist, in der die jeweilige Strahlquelle umläuft, die dem Detektor zugeordnet ist. Dabei ist ein jeweiliger Zentralstrahl der jeweiligen Strahlquelle auf den jeweiligen, dieser zugeordneten Detektor des jeweiligen Strahlquelle-Detektor-Paares ausgerichtet. Das Volumenbilderzeugungssystem ist zum Erzeugen der Abfolge von Volumenbildern des Gefäßsystems unter Berücksichtigung von ersten Vertrauenswerten der ersten Pixelwerte und/oder unter Berücksichtigung von zweiten Vertrauenswerten der zweiten Pixelwerte vorbereitet. Hierbei ist der Vertrauenswert eines Pixelwerts jeweils von einer pixelspezifischen Durchlauflänge abhängig, die ein Projektionsstrahl auf einem Weg durch Teile des Gefäßsystems von der ersten oder zweiten Strahlquelle zu einem pixelspezifischen Sensorelement des zugehörigen ersten oder zweiten Detektors durchläuft. Der jeweilige Vertrauenswert ist dabei umso größer, je weniger Teile des Gefäßsystems der jeweilige Projektionsstrahl durchlaufen hat, da dieser dann möglichst wenig geschwächt ist.

Das erfindungsgemäße Verfahren zum Erzeugen einer Abfolge von Volumenbildern eines Gefäßsystems umfasst folgende Handlungen. In einer ersten Handlung wird eine Abfolge von ersten Projektionsbilddatensätzen mit ersten Pixelwerten aus einem ersten Projektionswinkel erfasst. In einer zweiten Handlung wird eine Abfolge von zweiten Projektionsbilddatensätzen mit zweiten Pixelwerten aus einem zweiten Projektionswinkel erfasst. Hierbei unterscheidet sich der zweite Projektionswinkel von dem ersten Projektionswinkel. Die Abfolgen der ersten und zweiten Projektionsbilddatensätze werden während eines synchronen und rotationswinkelversetzten Umlaufs oder Teilumlaufs der beiden Strahlquelle-Detektor-Paare erfasst, wobei für beide Strahlquelle-Detektor-Paare gilt, dass der jeweilige Detektor in einer Rotationsebene umläuft, die zu der Rotationsebene beabstandet ist, in der die jeweilige Strahlquelle umläuft, die dem Detektor zugeordnet ist. Dabei ist ein jeweiliger Zentralstrahl der jeweiligen Strahlquelle auf den jeweiligen, dieser zugeordneten Detektor des jeweiligen Strahlquelle-Detektor-Paares ausgerichtet. In einer dritten Handlung wird die Abfolge von Volumenbildern unter Berücksichtigung von ersten Vertrauenswerten der ersten Pixelwerte und/oder unter Berücksichtigung von zweiten Vertrauenswerten der zweiten Pixelwerte erzeugt, wobei der Vertrauenswert eines Pixelwerts jeweils von einer pixelspezifischen Durchlauflänge abhängig ist, die ein Projektionsstrahl auf einem Weg durch Teile des Gefäßsystems von der ersten oder zweiten Strahlquelle zu einem pixelspezifischen Sensorelement des zugehörigen ersten oder zweiten Detektors durchläuft.

Die Tomografieanlage ist also dazu vorbereitet, die Abfolgen der ersten und zweiten Projektionsbilddatensätze während eines synchronen und rotationswinkelversetzten Umlaufs oder Teilumlaufs der beiden Strahlquelle-Detektor-Paare zu erfassen, wobei für beide Strahlquelle-Detektor-Paare gilt, dass der jeweilige Detektor in einer Rotationsebene umläuft, die zu der Rotationsebene beabstandet ist, in der die Strahlquelle umläuft, die dem Detektor zugeordnet ist. Durch eine Beabstandung der Rotationsebene des Detektors eines Strahlquelle-Detektor-Paars zu der Rotationsebene der Strahlquelle des Strahlquelle-Detektor-Paars kann erreicht werden, dass es innerhalb des rekonstruierbaren Volumens keine Ebene gibt, in der aus Sicht aller Voxel dieser Ebene mit der synchronen Rotation der Strahlquelle-Detektor-Paare um eine gemeinsame Orbitalachse nur höchstens zwei der drei Eulerwinkel variiert werden. Eine Variation aller drei Eulerwinkel für alle Voxel (mit Ausnahme solcher Voxel, durch die die Orbitalachse verläuft), fördert die Wahrscheinlichkeit dafür, dass für wenigstens einen der Projektionswinkel der Projektionsstrahl durch besonders wenige hintereinander liegende Gefäßabschnitte verläuft und somit dessen Pixelwert besonders aussagekräftig ist. Denn dann wird die verfügbare Quantisierungstiefe (von beispielsweise 14 Bit) für ein Minimum von durchstrahlten Gefäßabschnitten genutzt.

Ein Konzept der vorliegenden Erfindung kann darin gesehen werden, dass die Abfolge von Volumenbildern unter Berücksichtigung von ersten Vertrauenswerten der ersten Pixelwerte und/oder unter Berücksichtigung von zweiten Vertrauenswerten der zweiten Pixelwerte erzeugt wird, wobei der Vertrauenswert eines Pixelwerts jeweils von einer pixelspezifischen Durchlauflänge abhängig ist, die ein Projektionsstrahl auf einem Weg durch Teile des Gefäßsystems von der ersten oder zweiten Strahlquelle zu einem pixelspezifischen Sensorelement des zugehörigen ersten oder zweiten Detektors durchläuft. Jede dieser Maßnahmen stellt einen Beitrag dazu dar, ein Artefakt (Abbildungsuntreue) infolge eines Einbeziehens eines nicht ausreichend vertrauenswürdigen Pixelwerts zu vermeiden.

In einer ersten Ausführungsform ist das Volumenbilderzeugungssystem dazu vorbereitet, zum Ermitteln eines Voxelwerts eines Voxels einen gewichteten Mittelwert aus Pixelwerten von solchen ersten und zweiten Pixeln zu bilden, deren Projektionsstrahlen sich in dem Voxel schneiden, wobei der Vertrauenswert des ersten Pixelwerts als Gewicht des ersten Pixelwerts und der Vertrauenswert des zweiten Pixelwerts als Gewicht des zweiten Pixelwerts verwendet wird. Durch Einbeziehen von Pixelwerten entsprechend ihrer jeweiligen Vertrauenswürdigkeit (ihrer Vertrauenswerte) kann jeder Pixelwert entsprechend seiner Vertrauenswürdigkeit zum Berechnen von Voxelwerten solcher Voxel beitragen (genutzt werden), durch die ein Projektionsstrahl zum pixelspezifischen Sensorelement verläuft. Damit beeinflussen Pixelwerte den Voxelwert eines Voxels, durch das die Projektionsstrahlen zu den beiden Sensorelementen verlaufen, die den beiden Pixeln zugeordnet sind, dann in gleicher Weise, wenn die beiden Pixelwerte gleich vertrauenswürdig sind (also einen gleichen Vertrauenswert aufweisen). Außerdem beeinflusst dann ein Pixelwert den Voxelwert eines Voxels, durch das der Projektionsstrahl zu dem Sensorelement verläuft, der dem Pixel zugeordnet sind, gar nicht, wenn dem Pixelwert gar keine Vertrauenswürdigkeit beigemessen wird (er also einen Vertrauenswert von Null aufweist). Durch die vertrauenswertabhängige Gewichtung der Pixelwerte kann zwischen diesen beiden Extremen auch jede Zwischenstufe berücksichtigt werden (soweit die Quantisierungen der Vertrauenswerte und der Pixelwerte dies zulassen).

In einer zweiten Ausführungsform ist das Volumenbilderzeugungssystem dazu vorbereitet, zum Ermitteln eines Voxelwerts von einem Voxel einen Pixelwert eines ersten Pixels, dessen Projektionsstrahl sich in dem Voxel mit dem Projektionsstrahl eines zweiten Pixels schneidet, durch den zweiten Pixelwert des zweiten Pixels zu ersetzen, wenn der Vertrauenswert des ersten Pixelwerts einen ersten Schwellenwert unterschreitet. Alternativ oder zusätzlich kann das Volumenbilderzeugungssystem dazu vorbereitet sein, einen Pixelwert eines zweiten Pixels, dessen Projektionsstrahl sich in dem Voxel mit dem Projektionsstrahl eines ersten Pixels schneidet, durch den ersten Pixelwert des ersten Pixels zu ersetzen, wenn der Vertrauenswert des zweiten Pixelwerts den ersten Schwellenwert unterschreitet. Durch Unberücksichtigtlassen von solchen Pixelwerten, deren Vertrauenswerte den ersten Schwellenwert unterschreitet, können Zufälligkeiten im Volumenbild vermieden werden, die auf einem Einbeziehen von nicht ausreichend vertrauenswürdigen Pixelwerten beruhen.

In einer dritten Ausführungsform ist das Volumenbilderzeugungssystem dazu vorbereitet, zum Ermitteln eines Voxelwerts von einem Voxel unter Berücksichtigung von Pixelwerten von ersten und zweiten Pixeln, deren Projektionsstrahlen sich in dem Voxel schneiden, den Pixelwert des ersten Pixels durch eine zeitliche Interpolation und/oder eine Interpolation zwischen Pixelwerten von Pixeln zu ersetzen, die zu dem ersten Pixeln räumlich benachbart sind, wenn der Vertrauenswert des ersten Pixelwerts einen zweiten Schwellenwert unterschreitet. Alternativ oder zusätzlich kann das Volumenbilderzeugungssystem dazu vorbereitet sein, zum Ermitteln eines Voxelwerts den Pixelwert des zweiten Pixels durch eine zeitliche Interpolation und/oder eine Interpolation zwischen Pixelwerten von Pixeln zu ersetzen, die zu dem zweiten Pixel räumlich benachbart sind, wenn der Vertrauenswert des zweiten Pixelwerts den zweiten Schwellenwert unterschreitet. Besonders bevorzugt ist, wenn die zeitliche und/oder räumliche Interpolation zwischen Pixelwerten von zeitlich und/oder räumlich benachbarten Pixeln für Pixelwerte von solchen Paaren von ersten und zweiten Pixeln vorbereitet ist, deren beide Pixelwerte jeweils einen Vertrauenswert aufweisen, der einen vorgegebenen Schwellenwert (beispielsweise den ersten Schwellenwert) unterschreitet.

In einer weiteren Ausführungsform ist das Volumenbilderzeugungssystem dazu vorbereitet, zum Ermitteln eines Voxelwerts von einem Voxel Pixelwerte von ersten und zweiten Pixeln, deren Projektionsstrahlen sich in dem Voxel schneiden, arithmetisch zu mitteln. Dies Ausführungsform ist vergleichsweise einfach zu realisieren.

Die Abfolgen der ersten und zweiten Projektionsbilddatensätze könnten auch bei konstantem ersten und zweiten Projektionswinkel erfasst werden. Hierdurch kann ein mechanischer Aufwand für eine Rotation der beiden Strahlquelle-Detektor-Paare um eine Orbitalachse während des Erfassens der Abfolge von Projektionsbilddatensätzen eingespart werden. Außerdem vereinfacht sich das Volumenbilderzeugungssystem, wenn die Projektionsbilddatensätze für die Inphase in einem ersten Projektionswinkel aufgenommen werden, der konstant ist, und die Projektionsbilddatensätze für die Quadraturphase in einem zweiten Projektionswinkel aufgenommen werden, der ebenfalls konstant ist.

Zusätzlich kann die Tomografieanlage dazu vorbereitet sein, die Abfolgen der ersten und zweiten Projektionsbilddatensätze während eines synchronen und rotationswinkelversetzten Umlaufs oder Teilumlaufs beider Strahlquelle-Detektor-Paare in einer gemeinsamen Rotationsebene zu erfassen. Hierdurch kann gleichzeitig mit den Inphase-Quadratur-Aufnahmen ein Volumenbild aufgenommen werden, das allerdings keine (oder zumindest nicht dieselbe) zeitliche Auflösung wie die Inphase-Quadratur-Aufnahmen aufweist.

In einer vierten Ausführungsform ist das Volumenbilderzeugungssystem dazu vorbereitet, ein erstes dreidimensionales Maskenbild aus den Projektionsbilddatensätzen des synchronen und rotationswinkelversetzten Umlaufs oder Teilumlaufs beider Strahlquelle-Detektor-Paare zu gewinnen, indem aus den Projektionsbilddatensätzen oder den daraus rekonstruierten Volumenbilddaten solche Daten entfernt werden, die sich während des Umlaufs oder Teilumlaufs beider Strahlquelle-Detektor-Paare ändern. Hierdurch wird ein Zeitaufwand und eine Belastung des Patienten für einen getrennten Maskenlauf vermieden.

In einer fünften Ausführungsform ist das Volumenbilderzeugungssystem dazu vorbereitet, die Abfolge der Volumenbilder des Gefäßsystems unter Berücksichtigung eines zweiten dreidimensionalen Maskenbilds aus einem Maskenlauf zu gewinnen. Hierdurch kann in den Volumenbildern eine Darstellung von Details vermieden werden, die für eine mit der Tomografieanlage durchzuführende Untersuchungs- und/oder Behandlungsaufgabe ohne Relevanz sind. Dadurch, dass die Tomografieanlage selbst für eine Durchführung des Maskenlaufs vorbereitet ist, wird die Notwendigkeit der Bereitstellung einer weiteren Tomografieanlage für eine eigene, vorbereitende Untersuchung zum Erzeugen des Maskenbildes vermieden. Außerdem ist auch vorstellbar, dass Verlässlichkeit und/oder Qualität der Registrierung des Maskenbildes gefördert wird, wenn sowohl das Maskenbild als auch die Projektionsbilddatensätze der Füllphasen und/oder Ausflussphasen mit einer selben Tomografieanlage erzeugt werden.

Alternativ oder zusätzlich kann die Tomografieanlage dazu vorbereitet sein, die Abfolge der Volumenbilder des Gefäßsystems unter Berücksichtigung eines dritten dreidimensionalen Maskenbilds zu gewinnen, das aus einer externen Datenquelle bezogen wird. Hierdurch kann das Maskenbild mittels einer weiteren Tomografieanlage in einer eigenen, vorbereitenden Untersuchung erzeugt und zum Registrieren auf den Volumenbildern für die Füllphase und/oder Ausflussphase an die erfindungsgemäße Tomografieanlage übertragen werden. Diese Ausführungsform kann die erfindungsgemäße Tomografieanlage erheblich vereinfachen, weil sie für das erfindungsgemäße Erfassen der Projektionsbilddatensätze aus den beiden Projektionswinkeln und zum Erzeugen der Abfolge von Volumenbildern eines Gefäßsystems weder mechanisch noch elektrisch so aufwändig aufgebaut sein braucht, wie eine Biplan-Tomografieanlage zum Erzeugen eines dreidimensionalen Maskenbilds. Beispielsweise ist für das Inphase-Quadratur-Aufnahmeverfahren während der Füllphase und/oder während der Ausflussphase eine Rotation der beiden Aufnahmeanordnungen (um eine Orbitalachse) nicht erforderlich. Andererseits reicht eine Monoplan-Tomografieanlage für die Erzeugung eines dreidimensionalen Maskenbildes aus.

Eine zweckmäßige Weiterbildung sieht vor, dass das Volumenbilderzeugungssystem dazu vorbereitet ist, das erste und/oder das zweite und/oder das dritte dreidimensionale Maskenbild des Gefäßsystems als ein Begrenzungsbild für die Abfolge der Volumenbildern zu verwenden. Hierdurch können die vom Volumenbilderzeugungssystem erzeugten Volumenbilder von Inhalten freigehalten werden, die für eine mit der Tomografieanlage durchzuführende Untersuchung und/oder Behandlung nicht von Interesse sind, indem die Inhalte der Volumenbilder auf eine Darstellung von Füllzuständen von Teilen des Gefäßsystems beschränkt werden.

Insbesondere ist bevorzugt, wenn sich der zweite Projektionswinkel von dem ersten Projektionswinkel um 90° unterscheidet. Hierdurch wird das zweites Strahlquelle-Detektor-Paar zum Durchführen des Inphase-Quadratur-Aufnahmeverfahrens in bestmöglicher Weise genutzt.

Die Redewendung 'dazu vorbereitet' wird hier verwendet, um eine funktionelle Eigenschaft der Tomografieanlage zu definieren. Eine Beurteilung, ob die Tomografieanlage die jeweils beanspruchte funktionelle Eigenschaft aufweist, erfordert in der Regel eine bestimmungsgemäße Inbetriebnahme der Tomografieanlage. Die bloße Möglichkeit, eine Software in einer rechnergesteuerten Tomografieanlage eine weiterentwickelte Software nachzuladen, damit die rechnergesteuerte Tomografieanlage die beanspruchte funktionelle Eigenschaft aufweist, wird hier nicht als eine solche funktionelle Eigenschaft angesehen, für die die bestimmte Tomografieanlage vorbereitet ist. Mit anderen Worten, im Sinne der vorliegenden Erfindung gilt eine Tomografieanlage erst dann als für die funktionelle Eigenschaft vorbereitet, wenn eine Verwirklichung der funktionellen Eigenschaft nicht nur grundsätzlich besteht, sondern die Verwirklichung der funktionellen Eigenschaft von einem Entwickler, Hersteller, Importeur, Anbieter oder Betreiber nachweisbar in die Tat umgesetzt wird. Um zu beurteilen, ob eine solche Verwirklichung der funktionellen Eigenschaft vorliegt, kann auch ein Benutzerhandbuch, eine Angebotsunterlage oder eine Service- oder Entwicklungsunterlage der Tomografieanlage verwendet werden, sofern verifiziert wurde oder unterstellt werden kann, dass das dazu verwendete Dokument den tatsächlichen Aufbau und/oder die tatsächlichen funktionellen Eigenschaften der Tomografieanlage zutreffend beschreibt.

Die Erfindung ist anhand der beigefügten Zeichnungen näher erläutert, in denen zeigen:
- FIG 1: eine schematische perspektivische Ansicht eines Biplan-Tomografiegeräts mit einer Patientenauflage und zwei C-Bögen, die je ein Strahlquelle-Detektor-Paar aufweisen,
- FIG 2: eine schematische perspektivische Ansicht von Strahlengängen eines Biplan-Tomografiegeräts in einer Inphase-Quadratur-Anordnung,
- FIG 3: eine Abfolge von Volumenbildern in schematisch perspektivischer Ansicht aufgetragen entlang einer Zeitachse,
- FIG 4: eine Übersicht eines Datenflusses zwischen einer erfindungsgemäßen Tomografieanlage und einer weiteren Tomografieanlage,
- FIG 5: eine schematische Darstellung eines Verlaufs eines Projektionsstrahls, der durch mehrere hintereinander liegende Gefäßabschnitte eines Gefäßsystems verläuft,
- FIG 6: eine schematische perspektivische Ansicht von Strahlengängen zweier Ausführungsformen eines Biplan-Tomografiegeräts mit zwei Rotationsebenen, die zueinander beabstandet sind, und
- FIG 7: schematisch einen Ablauf eines Verfahrens zum Erzeugen einer Abfolge von Volumenbildern eines Gefäßsystems.

Die nachfolgend näher geschilderten Ausführungsbeispiele stellen bevorzugte Ausführungsformen der vorliegenden Erfindung dar.

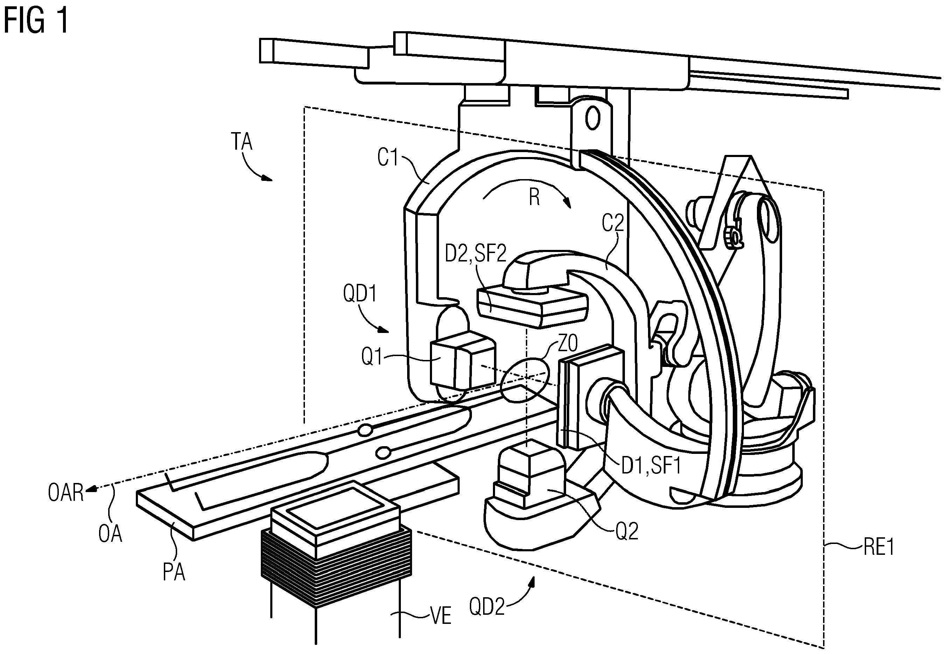

Die in FIG 1 gezeigte Biplan-Tomografieanlage TA weist einen ersten C1 und einen zweiten C2 C-Bogen sowie eine Patientenauflage PA und ein Volumenbilderzeugungssystem VE auf. An dem ersten C-Bogen C1 ist eine erste Strahlquelle Q1 und ein erster Detektor D1 befestigt. An dem zweiten C-Bogen C2 ist eine zweite Strahlquelle Q2 und ein zweiter Detektor D2 befestigt. Jeder der beiden Detektoren D1, D2 weist jeweils eine Sensorfläche SF1 bzw. SF2 auf.

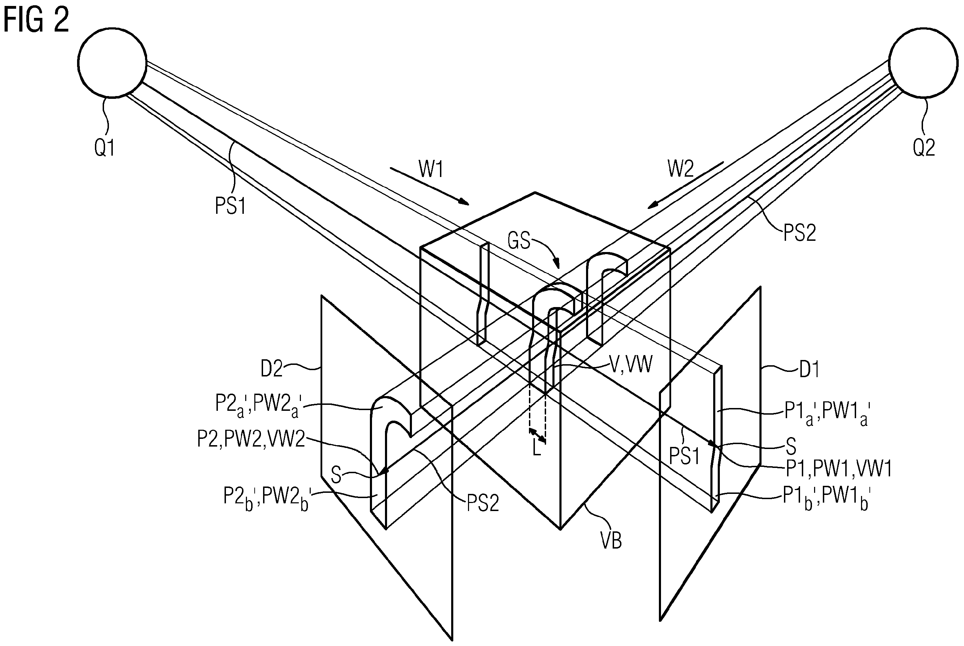

Das erste Strahlquelle-Detektor-Paar QD1 ist zum Erfassen einer Abfolge A1 von ersten Projektionsbilddatensätzen PB1 mit ersten Pixelwerten PW1 aus einem ersten Projektionswinkel W1, und ein zweites Strahlquelle-Detektor-Paar QD1 ist zum Erfassen aus einem zweiten Projektionswinkel W2 einer Abfolge A2 von zweiten Projektionsbilddatensätzen PB2 mit zweiten Pixelwerten PW2 vorbereitet, wobei sich die beiden Projektionswinkel W1, W2 unterscheiden (siehe FIG 2).

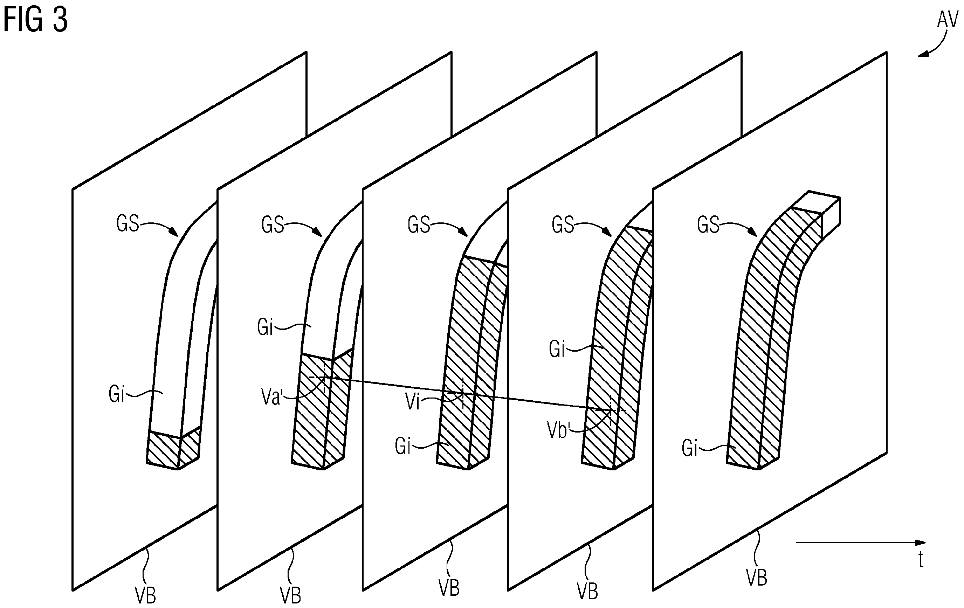

Das Volumenbilderzeugungssystem VE ist zum Erzeugen der Abfolge AV (siehe FIG 3) von Volumenbildern VB des Gefäßsystems GS unter Berücksichtigung von ersten Vertrauenswerten VW1 der ersten Pixelwerte PW1 und/oder unter Berücksichtigung von zweiten Vertrauenswerten VW2 der zweiten Pixelwerte PW2 vorbereitet. Hierbei ist der Vertrauenswert VW1 eines ersten Pixelwerts PW1 von einer pixelspezifischen Durchlauflänge L (siehe FIG 2) abhängig, die ein erster Projektionsstrahl PS1 auf einem Weg durch Teile Gi des Gefäßsystems GS von der ersten Strahlquelle Q1 zu einem pixelspezifischen Sensorelement S des zugehörigen ersten Detektors D1 durchläuft. Entsprechend ist der Vertrauenswert VW2 eines zweiten Pixelwerts PW2 von einer pixelspezifischen Durchlauflänge L abhängig, die ein zweiter Projektionsstrahl PS2 auf einem Weg durch Teile Gi des Gefäßsystems GS von der zweiten Strahlquelle Q2 zu einem pixelspezifischen Sensorelement S des zugehörigen zweiten Detektors D2 durchläuft.

In einer ersten Ausführungsform ist das Volumenbilderzeugungssystem VE dazu vorbereitet, zum Ermitteln eines Voxelwerts VW eines Voxels V einen gewichteten Mittelwert aus Pixelwerten von solchen ersten P1 und zweiten P2 Pixeln zu bilden, deren Projektionsstrahlen PS1, PS2 sich in dem Voxel V schneiden, wobei der Vertrauenswert VW1 des ersten Pixelwerts PW1 als Gewicht des ersten Pixelwerts PW1 und der Vertrauenswert VW1 des zweiten Pixelwerts PW2 als Gewicht des zweiten Pixelwerts PW2 verwendet wird. Durch Einbeziehen von Pixelwerten PW1, PW2 entsprechend ihrer jeweiligen Vertrauenswürdigkeit (also ihrer Vertrauenswerte) kann jeder Pixelwert PW1, PW2 entsprechend seiner Vertrauenswürdigkeit zum Berechnen von Voxelwerten VW solcher Voxel V beitragen (genutzt werden), durch die ein Projektionsstrahl PS1, PS2 zum pixelspezifischen Sensorelement S verläuft. Damit beeinflussen Pixelwerte PW1, PW2 den Voxelwert VW eines Voxels V, durch das die Projektionsstrahlen PS1, PS2 zu den beiden Sensorelementen S verlaufen, die den beiden Pixeln P1, P2 zugeordnet sind, dann in gleicher Weise, wenn die beiden Pixelwerte PW1, PW2 gleich vertrauenswürdig sind (also einen gleichen Vertrauenswert VW1, VW2 aufweisen). Außerdem beeinflusst dann ein Pixelwert PW1, PW2 den Voxelwert eines Voxels V, durch das der Projektionsstrahl PS1, PS2 zu dem Sensorelement S verläuft, das dem Pixel P1, P2 zugeordnet sind, gar nicht, wenn dem Pixelwert PW1, PW2 gar keine Vertrauenswürdigkeit beigemessen wird (es also einen Vertrauenswert von Null aufweist). Durch die vertrauenswertabhängige Gewichtung der Pixelwerte PW1, PW2 wird zwischen diesen beiden Extremen auch jede Zwischenstufe berücksichtigt (soweit die Quantisierungen der Vertrauenswerte VW1, VW2 und die Quantisierungen der Pixelwerte PW1, PW2 dies zulassen).

In einer zweiten Ausführungsform ist das Volumenbilderzeugungssystem VE dazu vorbereitet, zum Ermitteln eines Voxelwerts VW von einem Voxel V einen Pixelwert PW1 eines ersten Pixels P1, dessen Projektionsstrahl PS1 sich in dem Voxel V mit dem Projektionsstrahl PS2 eines zweiten Pixels P2 schneidet, durch den zweiten Pixelwert PW2 des zweiten Pixels P2 zu ersetzen, wenn der Vertrauenswert VW1 des ersten Pixelwerts PW1 einen ersten Schwellenwert unterschreitet. Alternativ oder zusätzlich kann das Volumenbilderzeugungssystem VE dazu vorbereitet sein, einen Pixelwert PW2 eines zweiten Pixels P2, dessen Projektionsstrahl PS2 sich in dem Voxel V mit dem Projektionsstrahl PS1 eines ersten Pixels P1 schneidet, durch den ersten Pixelwert PW1 des ersten Pixels P1 zu ersetzen, wenn der Vertrauenswert VW2 des zweiten Pixelwerts PW2 den ersten Schwellenwert unterschreitet. Durch Unberücksichtiglassen von solchen Pixelwerten PW1, PW2, deren Vertrauenswert VW1, VW2 den ersten Schwellenwert unterschreitet, können Zufälligkeiten im Volumenbild VB vermieden werden, die auf einem Einbeziehen von nicht ausreichend vertrauenswürdigen Pixelwerten PW1, PW2 beruhen.

Die FIG 2 zeigt eine Anordnung zum Aufnehmen einer ersten Abfolge A1 von ersten Projektionsbilddatensätzen PB1 und synchronen Aufnehmen einer zweiten Abfolge A2 von zweiten Projektionsbilddatensätzen PB2, wobei eine zweite Projektionsrichtung W2 zum Aufnehmen der zweiten Projektionsbilddatensätze PB2 senkrecht zu einer ersten Projektionsrichtung W1 zum Aufnehmen der ersten Projektionsbilddatensätze PB1 angeordnet ist.

Die FIG 3 zeigt eine Abfolge AV von Volumenbildern VB mit unterschiedlichen Füllzuständen eines Gefäßsystems GS über die Zeitachse t (die Schraffierung zeigt den jeweiligen Umfang der Füllung an).

In einer dritten Ausführungsform ist das Volumenbilderzeugungssystem dazu vorbereitet, zum Ermitteln eines Voxelwerts VW von einem Voxel V unter Berücksichtigung von Pixelwerten PW1, PW2 von ersten P1 und zweiten P2 Pixeln, deren Projektionsstrahlen PS1, PS2 sich in dem Voxel V schneiden, den Pixelwert PW1 des ersten Pixels P1 durch eine zeitliche Interpolation und/oder eine Interpolation zwischen Pixelwerten PW1' von Pixeln P1' zu ersetzen, die zum dem ersten Pixel P1 räumlich benachbart sind, wenn der Vertrauenswert VW1 des ersten Pixelwerts P1 einen zweiten Schwellenwert unterschreitet (siehe auch FIG 2). Hierbei ist besonders bevorzugt, wenn nur oder vorzugsweise nur mit solchen Pixelwerten PW' interpoliert wird, deren Vertrauenswert den zweiten Schwellenwert nicht unterschreitet.

Alternativ oder zusätzlich kann das Volumenbilderzeugungssystem VE dazu vorbereitet sein, zum Ermitteln eines Voxelwerts VW den Pixelwert PW2 des zweiten Pixels P2 durch eine zeitliche Interpolation und/oder eine Interpolation zwischen Pixelwerten PW2' von Pixeln P2' zu ersetzen, die zu dem zweiten Pixel P2 räumlich benachbart sind, wenn der Vertrauenswert VW2 des zweiten Pixelwerts PW2 den zweiten Schwellenwert unterschreitet. Hierbei ist besonders bevorzugt, wenn nur oder vorzugsweise nur mit solchen Pixelwerten PW' interpoliert wird, deren Vertrauenswert den zweiten Schwellenwert nicht unterschreitet.

Die FIG 3 zeigt ein zu interpolierendes Voxel Vi, das im Schnittpunkt der Projektionsstrahlen PS1, PS2 der Pixel P1, P2 liegt (siehe FIG 2). Entsprechend liegt ein erstes zeitlich und räumlich benachbarte Voxel Va' im Schnittpunkt von Projektionsstrahlen der Pixel P1a', P2a' und ein zweites räumlich benachbartes Voxel Vb' im Schnittpunkt von Projektionsstrahlen der Pixel P1b', P2b'. Das Voxel Va' ist ein erstes von zwei Voxeln Va', Vb', die zu dem zu interpolierenden Voxel Vi zeitlich und räumlich benachbart sind. Das Voxel Vb' ist ein zweites von zwei Voxeln Va', Vb', die zu dem zu interpolierenden Voxel Vi zeitlich und räumlich benachbart sind. Die Verbindungslinie zwischen den beiden zeitlich und räumlich benachbarten Voxeln Va' und Vb' ist eine Interpolationsgerade zwischen den Nachbarvoxeln Va', Vb' dar, deren Vertrauenswerte den zweiten Schwellenwert jeweils nicht unterschreiten.

Besonders bevorzugt ist, wenn die zeitliche und/oder räumliche Interpolation zwischen Pixelwerten von zeitlich und/oder räumlich benachbarten Pixeln für Pixelwerte PW1, PW2 von solchen Paaren von ersten P1 und zweiten Pixeln P2 vorbereitet ist, deren beide Pixelwerte PW1, PW2 jeweils einen Vertrauenswert VW1, VW2 aufweisen, der einen vorgegebenen Schwellenwert (beispielsweise den ersten Schwellenwert) unterschreitet.

In einer weiteren Ausführungsform ist das Volumenbilderzeugungssystem VE dazu vorbereitet, zum Ermitteln eines Voxelwerts VW von einem Voxel V Pixelwerte PW1, PW2 von ersten P1 und zweiten P2 Pixeln, deren Projektionsstrahlen PS1, PS2 sich in dem Voxel V schneiden, arithmetisch zu mitteln. Diese Ausführungsform ist vergleichsweise einfach zu realisieren.

In einer besonders bevorzugten Ausführungsform ist das Volumenbilderzeugungssystem VE dazu vorbereitet, ein erstes dreidimensionales Maskenbild M1 aus den Projektionsbilddatensätzen PB1, PB2 des synchronen und rotationswinkelversetzten Umlaufs oder Teilumlaufs beider Strahlquelle-Detektor-Paare QD1, QD2 zu ermitteln. Hierzu entfernt das Volumenbilderzeugungssystem VE aus den Projektionsbilddatensätzen PB1 oder den daraus rekonstruierten Volumenbilddaten solche Daten, die sich während des Umlaufs oder Teilumlaufs beider Strahlquelle-Detektor-Paare QD1, QD2 ändern (also beispielsweise vom Füllzustand oder Ausflusszustand des Gefäßsystems abhängig sind). Für diese Ausführungsform ist es zweckmäßig, wenn sich der synchrone und rotationswinkelversetzte Umlauf oder Teilumlauf beider Strahlquelle-Detektor-Paare QD1, QD2 mindestens über eine volle Füllphase oder mindestens über eine volle Ausflussphase erstreckt (während der die Abfolgen A1, A2 von Projektionsbilddatensätzen PB1, PB2 erfasst werden, beispielsweise mit einer Taktrate von 15 oder 30 Bildern pro Sekunde). Dieses Konzept der Gewinnung eines dreidimensionalen Maskenbildes M1 während einer Füllphase oder während einer Ausflussphase kann auch mit einer Monoplan-Tomografieanlage verwirklicht werden, die nur ein Strahlquelle-Detektor-Paar QD1 aufweist.

Alternativ oder zusätzlich kann die Tomografieanlage TA dazu vorbereitet sein, die Abfolge AV der Volumenbilder VB des Gefäßsystems GS unter Berücksichtigung eines zweiten dreidimensionalen Maskenbilds M2 aus einem Maskenlauf ML zu gewinnen. Hierdurch kann in den Volumenbildern VB eine Darstellung von Details (beispielsweise von Teilen eines Schädels) vermieden werden, die für eine mit der Tomografieanlage TA durchzuführende Untersuchungs- und/oder Behandlungsaufgabe ohne Relevanz sind. Dadurch, dass die Tomografieanlage TA selbst für eine Durchführung des Maskenlaufs ML vorbereitet ist, wird die Notwendigkeit der Bereitstellung einer weiteren Tomografieanlage TA für eine eigene, vorbereitende Untersuchung zum Erzeugen des Maskenbildes vermieden. Außerdem ist auch vorstellbar, dass Verlässlichkeit und/oder Qualität der Registrierung des Maskenbildes gefördert wird, wenn sowohl das Maskenbild als auch die Projektionsbilddatensätze PB1, PB2 während Füllphasen und/oder Ausflussphasen mit derselben Tomografieanlage TA erzeugt werden.

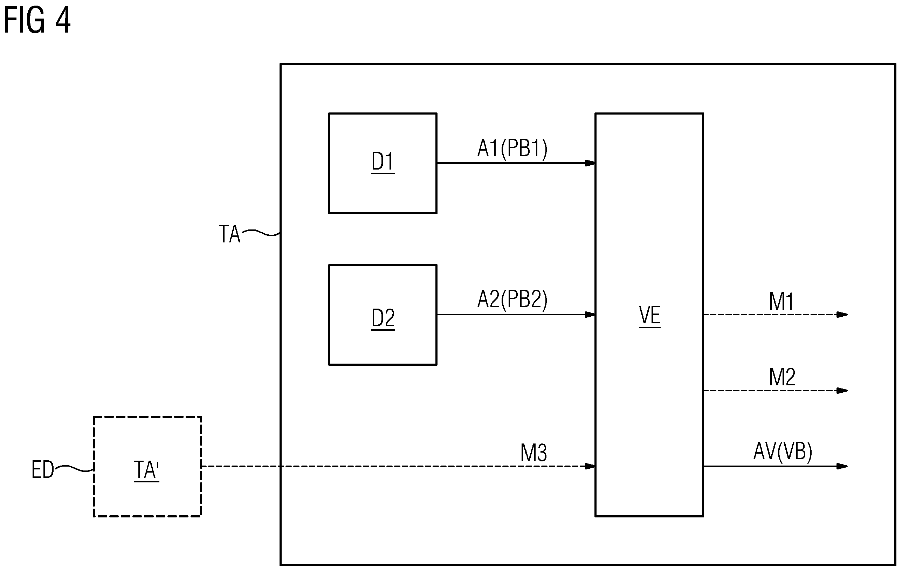

Wie FIG 4 zeigt, kann die Tomografieanlage TA alternativ oder zusätzlich dazu vorbereitet sein, die Abfolge AV der Volumenbilder VB des Gefäßsystems GS unter Berücksichtigung eines dritten dreidimensionalen Maskenbilds M3 zu gewinnen, das aus einer externen Datenquelle ED bezogen wird. Hierdurch kann das Maskenbild M3 mittels einer weiteren Tomografieanlage TA' in einer eigenen, vorbereitenden Untersuchung erzeugt und zum Registrieren auf den Volumenbildern VB für die Füllphase und/oder Ausflussphase an die erfindungsgemäße Tomografieanlage TA übertragen werden. Diese Ausführungsform kann die erfindungsgemäße Tomografieanlage TA erheblich vereinfachen, weil sie für das erfindungsgemäße Erfassen der Projektionsbilddatensätze PB1, PB2 aus den beiden Projektionswinkeln W1, W2 und zum Erzeugen der Abfolge AV von Volumenbildern VB eines Gefäßsystems GS weder mechanisch noch elektrisch so aufwändig aufgebaut sein braucht, wie eine Biplan-Tomografieanlage zum Erzeugen eines dreidimensionalen Maskenbilds. Insbesondere ist für das Inphase-Quadratur-Aufnahmeverfahren (während der Füllphase und/oder der Ausflussphase) eine Rotation R der beiden Aufnahmeanordnungen QD1, QD2 (um eine Orbitalachse OA) nicht erforderlich. Andererseits reicht eine Monoplan-Tomografieanlage TA' für die Erzeugung eines dreidimensionalen Maskenbildes M3 aus.

Eine zweckmäßige Weiterbildung sieht vor, dass das Volumenbilderzeugungssystem VE dazu vorbereitet ist, das erste M1 und/oder das zweite M2 und/oder das dritte M3 dreidimensionale Maskenbild des Gefäßsystems GS als ein Begrenzungsbild für die Abfolge AV der Volumenbildern VB zu verwenden. Hierdurch können die vom Volumenbilderzeugungssystem VE erzeugten Volumenbilder VB von Inhalten freigehalten werden, die für eine mit der Tomografieanlage TA durchzuführende Untersuchung und/oder Behandlung nicht von Interesse sind, indem die Inhalte der Volumenbilder VB auf eine Darstellung von Füllzuständen von Teilen Gi des Gefäßsystems GS beschränkt werden.

Insbesondere ist bevorzugt, wenn sich der zweite Projektionswinkel W2 von dem ersten Projektionswinkel W1 um 90° unterscheidet. Hierdurch wird das zweites Strahlquelle-Detektor-Paar QD2 zum Durchführen des Inphase-Quadratur-Aufnahmeverfahrens in bestmöglicher Weise genutzt.

Die Tomografieanlage TA kann auch dazu vorbereitet sein, die Abfolgen A1, A2 der ersten PB1 und zweiten PB2 Projektionsbilddatensätze bei konstantem ersten W1 und konstantem zweiten W2 Projektionswinkel zu erfassen. Hierdurch kann ein mechanischer Aufwand für eine Rotation R der beiden Strahlquelle-Detektor-Paare QD1, QD2 um eine Orbitalachse OA während des Erfassens der Abfolge A1, A2 von Projektionsbilddatensätzen PB1, PB2 eingespart werden. Außerdem vereinfacht sich das Volumenbilderzeugungssystem VE, wenn die ersten Projektionsbilddatensätze PB1 für die Inphase in einem ersten Projektionswinkel W1 aufgenommen werden, der konstant ist, und die zweiten Projektionsbilddatensätze PB2 für die Quadraturphase in einem zweiten Projektionswinkel W2 aufgenommen werden, der ebenfalls konstant ist.

Zusätzlich kann die Tomografieanlage TA dazu vorbereitet sein, die Abfolgen A1, A2 der ersten PB1 und zweiten PB2 Projektionsbilddatensätze während eines synchronen und rotationswinkelversetzten Umlaufs oder Teilumlaufs beider Strahlquelle-Detektor-Paare QD1, QD2 in einer gemeinsamen Rotationsebene RE1 zu erfassen. Hierdurch kann gleichzeitig mit den Inphase-Quadratur-Aufnahmen PB1, PB2 ein Volumenbild VB aufgenommen werden, das allerdings keine (oder zumindest nicht dieselbe) zeitliche Auflösung der Inphase-Quadratur-Aufnahmen PB1, PB2 aufweist.

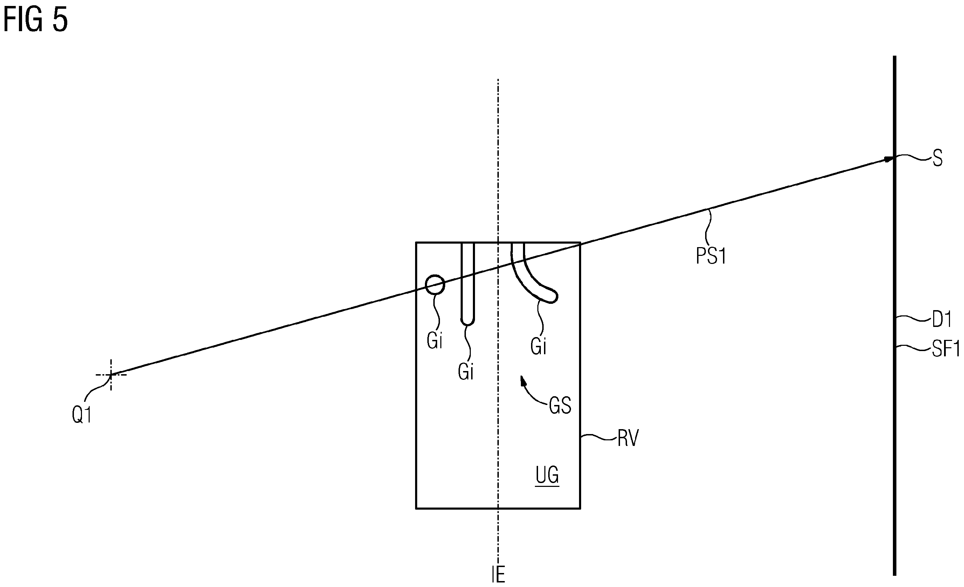

Die FIG 5 dient der Veranschaulichung des Problems der Überlagerung von Schattenbildern von Gefäßen Gi eines Gefäßsystems GS bei der Ermittlung eines Volumenbildes VB von einem rekonstruierbaren Volumen RV eines zu untersuchenden Objekts ZO. Die strichpunktierte Linie zeigt die Lage einer Isoebene IE. Es kann vorkommen, dass ein erster Projektionsstrahl PS1 mehrere Gefäße Gi durchläuft (ohne Beschränkung der Allgemeinheit wurden hier genau drei Gefäße gezeigt). Beim Durchlauf durch jedes dieser Gefäße Gi wird der Projektionsstrahl PS1 (infolge Absorption und eventuell auch Streuung) geschwächt. Die Schwächung der Intensität des Projektionsstrahls PS1 hängt sowohl von der Zusammensetzung des jeweiligen Gewebes Gi als auch von einer jeweiligen Durchlauflänge des Projektionsstrahls PS1 durch das jeweilige Gefäß Gi ab. Darüberhinaus wird die Intensität des Projektionsstrahls PS1 auch beim Durchlauf durch umgebendes Gewebe UG geschwächt, das die Gefäße Gi umgibt, die der Projektionsstrahl PS1 durchläuft.

In dem Volumenbild VB, das das Volumenbilderzeugungssystem VE zu ermitteln hat, ist für jedes vom Projektionsstrahl PS1 durchlaufende Gefäß Gi eine Information enthalten, wie stark das durchlaufende Gefäß Gi zur Schwächung der Intensität des Projektionsstrahls PS1 beiträgt. Die Stärke der Schwächung kann als Graustufe aufgefasst werden. Wenn die Schwächung der Intensität des Projektionsstrahls PS1 durch jedes einzelne Gefäß Gi und das umgebende Gewebe UG jeweils mit einem lineares Schwächungsmaß beziffert wird, errechnet sich die Gesamtschwächung der Intensität des Projektionsstrahls PS1 aus dem Produkt der Intensitätsschwächungen der einzelnen Gefäße Gi und des umgebenden Gewebes UG. Daraus folgt, dass eine Gesamtdämpfung der Intensität des Projektionsstrahls PS1 aus der Summe von Dämpfungen der einzelnen Gefäße Gi und des umgebenden Gewebes UG errechnet werden kann, wenn die Dämpfungen beziehungsweise die Gesamtdämpfung jeweils ein Logarithmus des linearen Schwächungsmaßes ist. Das Auflösungsvermögen hinsichtlich der Gesamtdämpfung (also die sogenannte Graustufenzahl) ist durch eine nutzbare Bittiefe der Analog-DigitalWandler des Detektors begrenzt (wobei die nutzbare Bittiefe zusätzlich auch durch Störeinflüsse wie Quantenrauschen begrenzt sein kann).

Beispielsweise sind mit einer Bittiefe von 12 Bit (4096 Intensitätswerte) höchstens 6 Gefäßabschnitte mit einer Diskretisierung von 4 Dämpfungswerten (2 Bit) oder höchstens 4 Gefäßabschnitte mit einer Diskretisierung von 8 Dämpfungswerten (3 Bit) auflösbar.

Aus diesem Grund ist es von Vorteil, wenn jedes Voxel V aus möglichst vielen Richtungen durchstrahlt wird, um dabei auch einmal eine Strahlrichtung (Blickrichtung durch das "Fadenknäuel" des Gefäßsystems) zu nutzen, in der nicht zu viele Gefäßabschnitte Gi liegen, sodass die wenigen oder der einzige Gefäßabschnitt Gi in dieser Strahlrichtung mit einer möglichst hohen Dynamik (Bittiefe) für sich alleine erfasst wird.

Das Ziel, jedes Voxel V aus möglichst vielen Richtungen zu durchstrahlen, wird für die Voxel V der Rotationsebene RE1 verfehlt, da die Voxel V der Rotationsebene RE1 zwar aus verschiedenen Umfangswinkeln, aber doch nur ausschließlich in Radialrichtung durchstrahlt werden. Mit anderen Worten: von den Eulerwinkeln wird dann nur der Rollwinkel variiert. Bei allen anderen Voxeln V (die nicht in der Rotationsebene liegen) führt die Rotation R auch zu einer voxelspezifischen Variation von Scherwinkel und Nickwinkel.

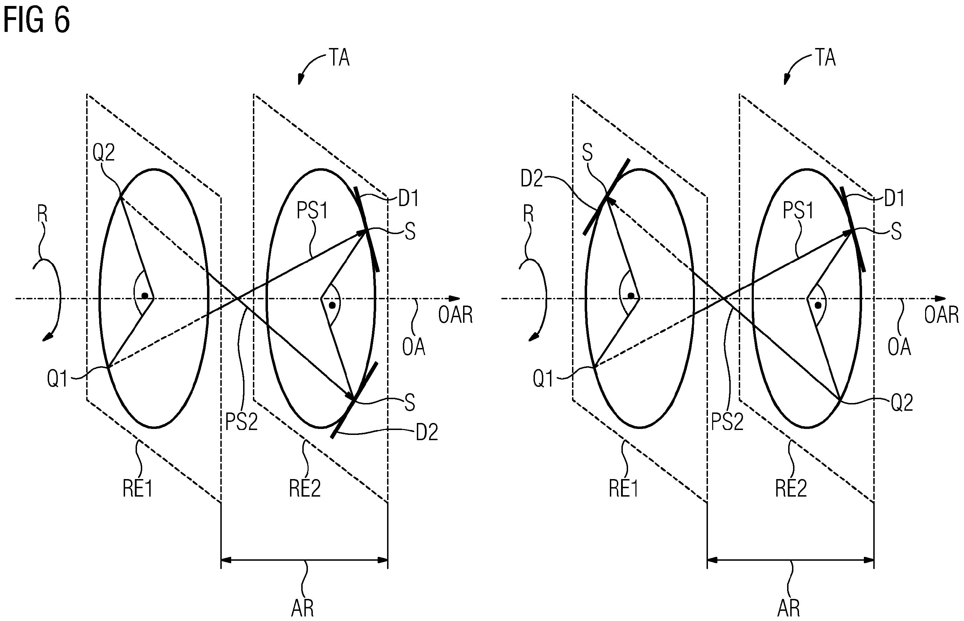

Die FIG 6 zeigt schematisch zwei Ausführungsvarianten einer Tomografieanlage TA, die das Überlappungsproblem dadurch mildert, dass die Rotationsebenen RE1, RE2 von Strahlquelle Q1, Q2 und Detektor D1, D2 beabstandet angeordnet sind. Mit ausreichendem Abstand AR der Rotationsebenen RE1, RE2 (und Ausrichtung des Zentralstrahls auf den jeweiligen Detektor D1, D2, in der anderen Rotationsebene) kann erzwungen werden, dass für jedes betrachtete Voxel V die Strahlrichtung durch das betrachtete Voxel V taumelt (also nicht in einer selben Ebene verharrt).

Dieses Konzept zur Verringerung des Überlappungsproblems kann auch mit einer Monoplan-Tomografieanlage realisiert werden, die nur eine Strahlquelle Q1 und nur einen Detektor D1 aufweist. Dabei kann es sich beispielsweise um die Tomografieanlage TA' handeln, mit der der Maskenlauf ML durchgeführt wird. Optional kann dieses Konzept mit dem vorher beschriebenen Konzept kombiniert werden, in welchem das Volumenbilderzeugungssystem VE dazu vorbereitet ist, das dreidimensionale Maskenbild M1 aus den Projektionsbilddatensätzen PB1 eines Umlaufs oder Teilumlaufs des Strahlquelle-Detektor-Paars QD1 zu ermitteln, wobei das Volumenbilderzeugungssystem VE aus den Projektionsbilddatensätzen PB1 oder den daraus rekonstruierten Volumenbilddaten solche Daten entfernt, die sich während des Umlaufs oder Teilumlaufs beider Strahlquelle-Detektor-Paare QD1, QD2 ändern (also beispielsweise solche Daten entfernt, die vom Füllzustand oder Ausflusszustand des Gefäßsystems GS abhängig sind).

In der Ausführungsvariante, die in der linken Skizze der FIG 6 dargestellt ist, läuft die zweite Strahlquelle Q2 in der ersten Rotationsebene RE1 um und der zweite Detektor D2 synchron in einer zweiten Rotationsebene RE2 um, die zu der ersten Rotationsebene RE1 beabstandet ist. In der Ausführungsvariante, die in der rechten Skizze der FIG 6 dargestellt ist, läuft die zweite Strahlquelle Q2 in der zweiten Rotationsebene RE2 um und der zweite Detektor D2 synchron in der ersten Rotationsebene RE1 um.

Durch die Beabstandung der Rotationsebene RE1 der Strahlquelle Q1 des ersten Strahlquelle-Detektor-Paars QD1 zu der Rotationsebene RE2 des Detektors D1 des ersten Strahlquelle-Detektor-Paars QD1 und durch die entsprechende Maßnahme für das zweite Strahlquelle-Detektor-Paar QD2 wird erreicht, dass es innerhalb des rekonstruierbaren Volumens RV keine Ebene gibt, in der aus Sicht irgendeines Voxels V dieser Ebene mit der synchronen Rotation R der Strahlquelle-Detektor-Paare QD1, QD2 um die gemeinsame Orbitalachse OA nur höchstens zwei der drei Eulerwinkel variiert werden. Eine Variation aller drei Eulerwinkel für alle Voxel V (mit Ausnahme solcher Voxel, durch die die Orbitalachse verläuft), fördert eine Wahrscheinlichkeit, dass während des Umlaufs bzw. Teilumlaufs der Projektionsstrahl PS1, PS2 für zumindest einen der Aufnahmewinkel durch besonders wenige hintereinander liegende Gefäßabschnitte Gi verläuft und somit der Pixelwert PW1, PW2 dieses Projektionsstrahls PS1, PS2 besonders aussagekräftig ist, weil dann die verfügbare Quantisierungstiefe (von beispielsweise 14 Bit) zur Ermittlung der durch Gefäßabschnitte Gi verursachten Dämpfung einer Intensität des Projektionsstrahls PS1 für ein Minimum von durchstrahlten Gefäßabschnitten Gi genutzt wird.

Der erste C-Bogen C1 kann beispielsweise eine angulare Rotation um eine Orbitalachse OA durchführen, während der zweite C-Bogen C2 eine orbitale Rotation R um dieselbe Orbitalachse OA ausführt. Bei der orbitalen Rotation R des zweiten C-Arms C2 bleibt die (gedachte) Ebene, in der sich der zweite C-Arm C2 befindet, unverändert. Bei der angularen Rotation R des ersten C-Arms C1 dreht sich der erste C-Arm C1 um eine Befestigungsachse BA1 des C-Arms. Grundsätzlich ist es auch möglich, einen synchronen Scan durchzuführen, in dem die C-Arme C1, C2 vor dem Scan so angeordnet sind, dass beide C-Arme C1, C2 beim Scan eine angulare Rotation R ausführen oder dass sie vor dem Scan so angeordnet sind, dass beide C-Arme C1, C2 beim Scan eine orbitale Rotation R ausführen.

Für die erfindungsgemäße Tomografieanlage TA und alle offenbarten Ausführungsformen derselben gilt, dass sie dazu vorbereitet sein kann, zum Rekonstruieren eines Volumenbildes VB der Abfolge AV von Volumenbildern VB oder des Maskenbildes M1, M2 ein iteratives Rekonstruktionsverfahren, insbesondere ein modellbasiertes, iteratives Rekonstruktionsverfahren, anzuwenden. Hierdurch kann (im Vergleich zu anderen Rekonstruktionsverfahren) bei gleicher Bildqualität eine Strahlendosis verringert werden oder bei unveränderter Strahlendosis die Bildqualität erhöht werden, indem ein Rauschanteil und/oder ein Anteil von Artefakten verringert wird und/oder eine Niedrigkontrast-Erkennbarkeit verbessert wird.

Bildgebende iterative Rekonstruktionsverfahren sind dem Fachmann bekannt, weshalb deren Arbeitsweise im Folgenden nur grob beschrieben wird. Als 'iterative Rekonstruktionsverfahren' werden konvergente, numerische Lösungsverfahren bezeichnet, in denen je Iterationsschritt ein zwei- oder dreidimensionales Bild (hier dreidimensionales Volumenbild VB) des durchleuchteten Objekts ZO geschätzt wird. Die geschätzten Bilder der einzelnen Iterationsschritte werden durch Vergleich zwischen Projektionsbilddatensätzen, die durch eine Simulation einer Vorwärtsprojektion mit der Schätzung (d.h. Abbildung durch das geschätzte dreidimensionalen Volumenbild) aus dem jeweils vorausgehenden Iterationsschritt gewonnen werden, und tatsächlich erfassten Projektionsbilddatensätzen geschätzt. Die Vorwärtsprojektion wird unter denjenigen Bestrahlungsbedingungen simuliert, unter denen die Projektionsbilddatensätze (Projektionsbilder) erfasst wurden. Nach einigen vorausgehenden Iterationsschritten ist die Schätzung des dreidimensionalen Volumenbilds VB des durchleuchteten Objekts ZO so gut, dass dessen simulierte Vorwärtsprojektion nur noch geringfügig von den tatsächlich erfassten Projektionsbilddatensätzen (Projektionsbildern) abweicht und folglich das geschätzte dreidimensionale Volumenbild VB das zu untersuchende reale Objekt sehr gut wiedergibt. Voraussetzung hierfür ist eine Konvergenz des gewählten Schätzverfahrens. Unter einem modellbasierten, iterativen Rekonstruktionsverfahren wird ein iteratives Rekonstruktionsverfahren verstanden, das Vorausprojektionen realitätsnäher simuliert, indem es zusätzliche Betriebsparameter (beispielsweise Strahlweite, Winkelverschmierung) und/oder an sich unerwünschte physikalische Effekte (beispielsweise Strahlaufhärtung) berücksichtigt.

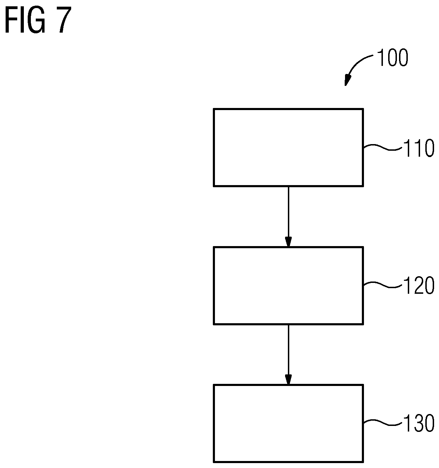

Das in FIG 7 dargestellte Verfahren 100 zum Erzeugen einer Abfolge AV von Volumenbildern VB eines Gefäßsystems GS umfasst folgende Handlungen. In einer ersten Handlung 110 wird eine Abfolge A1 von ersten Projektionsbilddatensätzen PB1 mit ersten Pixelwerten PW1 aus einem ersten Projektionswinkel W1 erfasst. In einer zweiten Handlung 120 wird eine Abfolge A2 von zweiten Projektionsbilddatensätzen PB1 mit zweiten Pixelwerten PW2 aus einem zweiten Projektionswinkel W2 erfasst, wobei sich der zweite Projektionswinkel W2 von dem ersten Projektionswinkel W1 unterscheidet. In einer dritten Handlung 130 wird die Abfolge AV von Volumenbildern VB unter Berücksichtigung von ersten Vertrauenswerten VW1 der ersten Pixelwerte PW1 und/oder unter Berücksichtigung von zweiten Vertrauenswerten VW2 der zweiten Pixelwerte PW2 erzeugt, wobei der Vertrauenswert VW1, VW2 eines Pixelwertes PW1, PW2 jeweils von einer pixelspezifischen Durchlauflänge L abhängig ist, die ein Projektionsstrahl P1, P2 auf einem Weg durch Teile Gi des Gefäßsystems GS von der ersten Q1 oder zweiten Q2 Strahlquelle zu einem pixelspezifischen Sensorelement S des zugehörigen ersten D1 oder zweiten D2 Detektors durchläuft.

Die Erfindung betrifft eine Tomografieanlage TA, die Folgendes aufweist: ein erstes QD1 und ein zweites QD2 Strahlquelle-Detektor-Paar zum Erfassen je einer Abfolge A1, A2 von Projektionsbilddatensätzen PB1, PB2 aus je einem Projektionswinkel W1, W2 sowie ein Volumenbilderzeugungssystem VE zum Erzeugen einer Abfolge AV von Volumenbildern VB eines Gefäßsystems GS unter Berücksichtigung von ersten Vertrauenswerten VW1 der ersten Pixelwerte PW1 und/oder unter Berücksichtigung von zweiten Vertrauenswerten VW2 der zweiten Pixelwerte PW2. Der Vertrauenswert VW1, VW2 eines Pixelwerts PW1, PW2 ist jeweils von einer pixelspezifischen Durchlauflänge L abhängig, die ein Projektionsstrahl PS1, PS2 auf einem Weg durch Teile Gi des Gefäßsystems GS von der ersten Q1 oder zweiten Q2 Strahlquelle zu einem pixelspezifischen Sensorelement S des zugehörigen ersten D1 oder zweiten D2 Detektors durchläuft.

Ein Konzept der vorliegenden Erfindung besteht in einem Berücksichtigen von pixelspezifischen Vertrauenswerten VW1, VW2, die von einer jeweiligen pixelspezifischen Durchlauflänge L abhängig sind, die ein pixelspezifischer Projektionsstrahl PS1, PS2 auf einem Weg durch Teile Gi des Gefäßsystems GS zum pixelspezifischen Sensorelement S durchläuft.

## Patentansprüche

1. Tomografieanlage (TA) aufweisend:
- ein erstes Strahlquelle-Detektor-Paar (QD1) zum Erfassen einer Abfolge (A1) von ersten Projektionsbilddatensätzen (PB1) mit ersten Pixelwerten (PW1) aus einem ersten Projektionswinkel (W1);
- ein zweites Strahlquelle-Detektor-Paar (QD2) zum Erfassen aus einem zweiten Projektionswinkel (W2) einer Abfolge (A2) von zweiten Projektionsbilddatensätzen (PB2) mit zweiten Pixelwerten (PW2), wobei sich die beiden Projektionswinkel (W1, W2) unterscheiden; und wobei
- die Tomografieanlage (TA) dazu eingerichtet ist, die Abfolgen (A1, A2) der ersten (PB1) und zweiten (PB2) Projektionsbilddatensätze während eines synchronen und rotationswinkelversetzten Umlaufs oder Teilumlaufs der beiden Strahlquelle-Detektor-Paare (QD1, QD2) zu erfassen, wobei für beide Strahlquelle-Detektor-Paare (QD1, QD2) gilt, dass der jeweilige Detektor (D1, D2) in einer Rotationsebene (RE1, RE2) umläuft, die zu der Rotationsebene (RE1, RE2) beabstandet ist, in der die Strahlquelle (Q1, Q2) umläuft, die dem Detektor (D1, D2) zugeordnet ist, wobei ein jeweiliger Zentralstrahl der jeweiligen Strahlquelle (Q1, Q2) auf den jeweiligen, dieser zugeordneten Detektor (D1, D2) des jeweiligen Strahlquelle-Detektor-Paares (QD1, QD2) ausgerichtet ist; und
- ein Volumenbilderzeugungssystem (VE) zum Erzeugen einer Abfolge (AV) von Volumenbildern (VB) eines Gefäßsystems (GS) unter Berücksichtigung von ersten Vertrauenswerten (VW1) als Gewicht der ersten Pixelwerte (PW1) und/oder unter Berücksichtigung von zweiten Vertrauenswerten (VW2) als Gewicht der zweiten Pixelwerte (PW2), wobei der Vertrauenswert (VW1, VW2) eines Pixelwerts (PW1, PW2) jeweils von einer pixelspezifischen Durchlauflänge (L) abhängig ist, die ein Projektionsstrahl (PS1, PS2) durch Teile (Gi) des Gefäßsystems (GS) auf einem Weg von der ersten (Q1) oder zweiten (Q2) Strahlquelle zu einem pixelspezifischen Sensorelement (S) des zugehörigen ersten (D1) oder zweiten (D2) Detektors durchläuft und wobei der Vertrauenswert (VW1, VW2) umso größer ist, je weniger Teile (Gi) des Gefäßsystems (GS) der jeweilige Projektionsstrahl (PS1, PS2) durchlaufen hat.

2. Tomografieanlage (TA) nach dem vorhergehenden Anspruch, **dadurch gekennzeichnet, dass** das Volumenbilderzeugungssystem (VE) dazu eingerichtet ist, zum Ermitteln eines Voxelwerts (VW) eines Voxels (V) einen gewichteten Mittelwert aus Pixelwerten (PW1, PW2) von solchen ersten (P1) und zweiten (P2) Pixeln zu bilden, deren Projektionsstrahlen (PS1, PS2) sich in dem Voxel (V) schneiden.

3. Tomografieanlage (TA) nach einem der vorhergehenden Ansprüche, **dadurch gekennzeichnet, dass**
das Volumenbilderzeugungssystem (VE) dazu eingerichtet ist, zum Ermitteln eines Voxelwerts (VW) von einem Voxel (V) einen Pixelwert (PW1) eines ersten Pixels (P1), dessen Projektionsstrahl (PS1) sich in dem Voxel (V) mit dem Projektionsstrahl (PS2) eines zweiten Pixels (P2) schneidet, durch den zweiten Pixelwert (PW2) des zweiten Pixels (P2) zu ersetzen, wenn der Vertrauenswert (VW1) des ersten Pixelwerts (PW1) einen ersten Schwellenwert unterschreitet, und/oder
dass das Volumenbilderzeugungssystem (VE) dazu vorbereitet ist, einen Pixelwert (PW2) eines zweiten Pixels (P2), dessen Projektionsstrahl (PS2) sich in dem Voxel (V) mit dem Projektionsstrahl (PS1) eines ersten Pixels (P1) schneidet, durch den ersten Pixelwert (PW1) des ersten Pixels (P1) zu ersetzen, wenn der Vertrauenswert (VW2) des zweiten Pixelwerts (PW2) den ersten Schwellenwert unterschreitet.

4. Tomografieanlage (TA) nach einem der vorhergehenden Ansprüche, **dadurch gekennzeichnet, dass** das Volumenbilderzeugungssystem (VE) dazu eingerichtet ist, zum Ermitteln eines Voxelwerts (VW) von einem Voxel (V) unter Berücksichtigung von Pixelwerten (PW1, PW2) von ersten (P1) und zweiten (P2) Pixeln, deren Projektionsstrahlen (PS1, PS2) sich in dem Voxel (V) schneiden, den Pixelwert (PW1) des ersten Pixels (P1) durch eine zeitliche Interpolation und/oder eine Interpolation zwischen Pixelwerten (PW1') von Pixeln (P1') zu ersetzen, die zu dem ersten Pixel (P1) räumlich benachbart sind, wenn der Vertrauenswert (VW1) des ersten Pixelwerts (PW1) einen zweiten Schwellenwert (SW2) unterschreitet, und/oder
den Pixelwert (PW2) des zweiten Pixels (P2) durch eine zeitliche Interpolation und/oder eine Interpolation zwischen Pixelwerten (PW2') von Pixeln (P2') zu ersetzen, die zu dem zweiten Pixel (P2) räumlich benachbart sind, wenn der Vertrauenswert (VW2) des zweiten Pixelwerts (PW2) den zweiten Schwellenwert (SW2) unterschreitet.

5. Tomografieanlage (TA) nach einem der vorhergehenden Ansprüche, **dadurch gekennzeichnet, dass** das Volumenbilderzeugungssystem (VE) dazu eingerichtet ist, ein erstes dreidimensionales Maskenbild (M1) aus den Projektionsbilddatensätzen (PB1, PB2) des synchronen und rotationswinkelversetzten Umlaufs oder Teilumlaufs beider Strahlquelle-Detektor-Paare (QD1, QD2) zu gewinnen, indem aus den Projektionsbilddatensätzen (PB1, PB2) oder den daraus rekonstruierten Volumenbilddaten solche Daten entfernt werden, die sich während des Umlaufs oder Teilumlaufs beider Strahlquelle-Detektor-Paare (QD1, QD2) ändern.

6. Tomografieanlage (TA) nach einem der vorhergehenden Ansprüche, **dadurch gekennzeichnet, dass** das Volumenbilderzeugungssystem (VE) dazu eingerichtet ist, die Abfolge (AV) der Volumenbilder (VB) des Gefäßsystems (GS) unter Berücksichtigung eines zweiten dreidimensionalen Maskenbilds (M2) aus einem Maskenlauf (ML) zu gewinnen und/oder unter Berücksichtigung eines dritten dreidimensionalen Maskenbilds (M3) zu gewinnen, das aus einer externen Datenquelle (ED) bezogen wird.

7. Tomografieanlage (TA) nach Anspruch 5 oder 6, **dadurch gekennzeichnet, dass** das Volumenbilderzeugungssystem (VE) dazu eingerichtet ist, das erste (M1) und/oder das zweite (M2) und/oder das dritte (M3) dreidimensionale Maskenbild des Gefäßsystems (GS) als ein Begrenzungsbild für die Abfolge (AV) der Volumenbilder (VB) zu verwenden.

8. Tomografieanlage (TA) nach einem der vorhergehenden Ansprüche, **dadurch gekennzeichnet, dass** sich der zweite Projektionswinkel (PW2) von dem ersten Projektionswinkel (PW1) um 90° unterscheidet.

9. Verfahren (100) zum Erzeugen einer Abfolge (AV) von Volumenbildern (VB) eines Gefäßsystems (GS), wobei das Verfahren (100) Folgendes umfasst:
- Erfassen (110) einer Abfolge (A1) von ersten Projektionsbilddatensätzen (PB1) mit ersten Pixelwerten (PW1) aus einem ersten Projektionswinkel (W1);
- Erfassen (120) einer Abfolge (A2) von zweiten Projektionsbilddatensätzen (PB2) mit zweiten Pixelwerten (PW2) aus einem zweiten Projektionswinkel (W2), wobei sich der zweite Projektionswinkel (W2) von dem ersten Projektionswinkel (W1) unterscheidet; wobei
- die Abfolgen (A1, A2) der ersten (PB1) und zweiten (PB2) Projektionsbilddatensätze während eines synchronen und rotationswinkelversetzten Umlaufs oder Teilumlaufs der beiden Strahlquelle-Detektor-Paare (QD1, QD2) erfasst werden, wobei für beide Strahlquelle-Detektor-Paare (QD1, QD2) gilt, dass der jeweilige Detektor (D1, D2) in einer Rotationsebene (RE1, RE2) umläuft, die zu der Rotationsebene (RE1, RE2) beabstandet ist, in der die Strahlquelle (Q1, Q2) umläuft, die dem Detektor (D1, D2) zugeordnet ist, wobei ein jeweiliger Zentralstrahl der jeweiligen Strahlquelle (Q1, Q2) auf den jeweiligen, dieser zugeordneten Detektor (D1, D2) des jeweiligen Strahlquelle-Detektor-Paares (QD1, QD2) ausgerichtet ist;
- Erzeugen (130) der Abfolge (AV) von Volumenbildern (VB) unter Berücksichtigung von ersten Vertrauenswerten (VW1) als Gewicht der ersten Pixelwerte (PW1) und/oder unter Berücksichtigung von zweiten Vertrauenswerten (VW2) als Gewicht der zweiten Pixelwerte (PW2), wobei der Vertrauenswert (VW1, VW2) eines Pixelwertes (PW1, PW2) jeweils von einer pixelspezifischen Durchlauflänge (L) abhängig ist, die ein Projektionsstrahl (PS1, PS2) durch Teile (Gi) des Gefäßsystems (GS) auf einem Weg von der ersten (Q1) oder zweiten (Q2) Strahlquelle zu einem pixelspezifischen Sensorelement (S) des zugehörigen ersten (D1) oder zweiten (D2) Detektors durchläuft, und wobei der jeweilige Vertrauenswert (VW1, VW2) umso größer ist, je weniger Teile (Gi) des Gefäßsystems (GS) der jeweilige Projektionsstrahl (PS1, PS2) durchlaufen hat.

## Claims

1. Tomography system (TA) comprising:
- a first beam source-detector pair (QD1) for capturing a series (A1) of first projection image data sets (PB1) containing first pixel values (PW1) from a first projection angle (W1);
- a second beam source-detector pair (QD2) for capturing a series (A2) of second projection image data sets (PB2) containing second pixel values (PW2) from a second projection angle (W2), wherein the two projection angles (W1, W2) are different from each other; and wherein
- the tomography system (TA) is prepared for capturing the series (A1, A2) of first (PB1) and second (PB2) projection image data sets during a synchronous and rotation-angle-offset revolution or partial revolution of the two beam source-detector pairs (QD1, QD2), wherein it is applicable to both beam source-detector pairs (QD1, QD2) that the respective detector (D1, D2) revolves in a plane of rotation (RE1, RE2) which is spaced apart at a distance from the plane of rotation (RE1, RE2) in which the beam source (Q1, Q2) that is assigned to the detector (D1, D2) revolves, wherein a respective central beam of the respective beam source (Q1, Q2) is oriented towards the respective detector (D1, D2) assigned thereto of the respective beam source-detector pair (QD1, QD2); and
- a volume image production system (VE) for producing a series (AV) of volume images (VB) of a vascular system (GS) taking into account first confidence values (VW1) as a weight of the first pixel values (PW1) and/or taking into account second confidence values (VW2) as a weight of the second pixel values (PW2), wherein the confidence value (VW1, VW2) of a pixel value (PW1, PW2) is dependent in each case on a pixel-specific traversing length (L) that a projection beam (PS1, PS2) traverses through parts (Gi) of the vascular system (GS) on a path from the first (Q1) or second (Q2) beam source to a pixel-specific sensor element (S) of the associated first (D1) or second (D2) detector and wherein the confidence value (VW1, VW2) is greater, the fewer parts (Gi) of the vascular system (GS) have been traversed by the respective projection beam (PS1, PS2).

2. Tomography system (TA) according to the preceding claim, **characterised in that** the volume image production system (VE) is prepared for forming a weighted average value from pixel values (PW1, PW2) of such first (P1) and second (P2) pixels whose projection beams (PS1, PS2) intersect in a voxel (V) in order to determine a voxel value (VW) of the voxel (V).

3. Tomography system (TA) according to one of the preceding claims, **characterised in that**
the volume image production system (VE) is prepared for replacing a pixel value (PW1) of a first pixel (P1) whose projection beam (PS1) intersects in a voxel (V) with the projection beam (PS2) of a second pixel (P2) by the second pixel value (PW2) of the second pixel (P2) if the confidence value (VW1) of the first pixel value (PW1) falls below a first threshold value in order to determine a voxel value (VW) of the voxel (V), and/or
the volume image production system (VE) is prepared for replacing a pixel value (PW2) of a second pixel (P2) whose projection beam (PS2) intersects in the voxel (V) with the projection beam (PS1) of a first pixel (P1) by the first pixel value (PW1) of the first pixel (P1) if the confidence value (VW2) of the second pixel value (PW2) falls below the first threshold value.

4. Tomography system (TA) according to one of the preceding claims, **characterised in that** the volume image production system (VE) is prepared for replacing the pixel value (PW1) of the first pixel (P1) by a temporal interpolation and/or an interpolation between pixel values (PW1') of pixels (P1') that are spatially adjacent to the first pixel (P1) if the confidence value (VW1) of the first pixel value (PW1) falls below a second threshold value (SW2) in order to determine a voxel value (VW) of a voxel (V) taking into account pixel values (PW1, PW2) of first (P1) and second (P2) pixels whose projection beams (PS1, PS2) intersect in the voxel (V), and/or for replacing the pixel value (PW2) of the second pixel (P2) by a temporal interpolation and/or an interpolation between pixel values (PW2') of pixels (P2') that are spatially adjacent to the second pixel (P2) if the confidence value (VW2) of the second pixel value (PW2) falls below the second threshold value (SW2).

5. Tomography system (TA) according to one of the preceding claims, **characterised in that** the volume image production system (VE) is prepared for obtaining a first three-dimensional mask image (M1) from the projection image data sets (PB1, PB2) of the synchronous and rotation-angle-offset revolution or partial revolution of the two beam source-detector pairs (QD1, QD2) by removing, from the projection image data sets (PB1, PB2) or the volume image data reconstructed therefrom, such data that changes during the revolution or partial revolution of the two beam source-detector pairs (QD1, QD2).

6. Tomography system (TA) according to one of the preceding claims, **characterised in that** the volume image production system (VE) is prepared for obtaining the series (AV) of volume images (VB) of the vascular system (GS) taking into account a second three-dimensional mask image (M2) from a mask run (M1) and/or taking into account a third three-dimensional mask image (M3) that is obtained from an external data source (ED) .

7. Tomography system (TA) according to claim 5 or 6, **characterised in that** the volume image production system (VE) is prepared for using the first (M1) and/or the second (M2) and/or the third (M3) three-dimensional mask image of the vascular system (GS) as a boundary image for the series (AV) of volume images (VB).

8. Tomography system (TA) according to one of the preceding claims, **characterised in that** the second projection angle (PW2) is offset by 90° from the first projection angle (PW1).

9. Method (100) for producing a series (AV) of volume images (VB) of a vascular system (GS), wherein the method (100) comprises the following:
- capturing (110) a series (A1) of first projection image data sets (PB1) containing first pixel values (PW1) from a first projection angle (W1);
- capturing (120) a series (A2) of second projection image data sets (PB2) containing second pixel values (PW2) from a second projection angle (W2), wherein the second projection angle (W2) is different from the first projection angle (W1); wherein
- the series (A1, A2) of the first (PB1) and second (PB2) projection image data sets are captured during a synchronous and rotation-angle-offset revolution or partial revolution of the two beam source-detector pairs (QD1, QD2), wherein it is applicable to both beam source-detector pairs (QD1, QD2) that the respective detector (D1, D2) revolves in a plane of rotation (RE1, RE2) which is spaced apart at a distance from the plane of rotation (RE1, RE2) in which the beam source (Q1, Q2) that is assigned to the detector (D1, D2) revolves, wherein a respective central beam of the respective beam source (Q1, Q2) is oriented towards the respective detector (D1, D2) assigned thereto of the respective beam source-detector pair (QD1, QD2);
- producing (130) the series (AV) of volume images (VB) taking into account first confidence values (VW1) as a weight of the first pixel values (PW1) and/or taking into account second confidence values (VW2) as a weight of the second pixel values (PW2), wherein the confidence value (VW1, VW2) of a pixel value (PW1, PW2) is dependent in each case on a pixel-specific traversing length (L) that a projection beam (PS1, PS2) traverses through parts (Gi) of the vascular system (GS) on a path from the first (Q1) or second (Q2) beam source to a pixel-specific sensor element (S) of the associated first (D1) or second (D2) detector, and wherein the respective confidence value (VW1, VW2) is greater, the fewer parts (Gi) of the vascular system (GS) have been traversed by the respective projection beam (PS1, PS2).

## Revendications

1. Installation (TA) de tomographie, comportant :
- une première paire (QD1) source de rayonnement-détecteur, pour détecter, à partir d'un premier angle (W1) de projection, une succession (A1) de premiers jeux (PB1) de données d'image de projection, ayant des premières valeurs (PW1) de pixel ;
- une deuxième paire (QD2) source de rayonnement-détecteur, pour détecter, à partir d'un deuxième angle (W2) de projection, une succession (A2) de deuxièmes jeux (PB2) de données d'image de projection, ayant des deuxièmes valeurs (PW2) de pixel, les deux angles (W1, W2) de projection étant différents ; et dans laquelle
- l'installation (TA) de tomographie est conçue pour détecter les successions (A1, A2) des premiers (PB1) et deuxièmes (PB2) jeux d'image de projection pendant un tour ou un tour partiel, en synchronisme et décalés en angle de rotation, des deux paires (QD1, QD2) source de rayonnement-détecteur, dans laquelle, pour les deux paires (QD1, QD2) source de rayonnement-détecteur, le détecteur (DA, D2) respectif tourne dans un plan (RE1, RE2) de rotation, qui est à distance du plan (RE1, RE2) de rotation, dans lequel tourne la source (Q1, Q2) de rayonnement associée au détecteur (D1, D2) un rayon central de la source (Q1, Q2) de rayonnement étant dirigée sur le détecteur (D1, D2), qui lui est associé, de la paire (QD1, QD2) source de rayonnement-détecteur respective ; et
- un système (VE) de production d'image en volume pour produire une succession (AV) d'images (VB) en volume d'un système (GS) de vaisseau, en tenant compte de premières valeurs (VW1) de confiance, comme poids des premières valeurs (PW1) de pixel, et/ou en tenant compte de deuxièmes valeurs (VW2) de confiance, comme poids des deuxièmes valeurs (PW2) de pixel, la valeur (VW1, VW2) de confiance d'une valeur (PW1, PW2) de pixel dépendant d'une longueur (L) de parcours, spécifique au pixel, qu'un rayon (PS1, PS2) de projection parcourt dans des parties (Gi) du système (GS) de vaisseau, sur un chemin allant de la première (Q1) ou de la deuxième (Q2) source de rayonnement à un élément (S) de capteur, spécifique au pixel, du premier (D1) ou du deuxième (D2) détecteur associé et la valeur (VW1, VW2) de confiance étant d'autant plus grande que le rayon (PS1, PS2) de projection a parcouru moins de parties (Gi) du système (GS) de vaisseau.

2. Installation (TA) de tomographie suivant la revendication précédente, **caractérisée en ce que** le système (VE) de production d'image en volume est conçu, pour la détermination d'une valeur (VW) d'un voxel (V), pour former une moyenne pondérée des valeurs (PW1, PW2) de pixel de tels premier (P1) et deuxième (P2) pixel, dont les rayons (PS1, PS2) de projection se coupent dans le voxel (V).

3. Installation (TA) de tomographie suivant l'une des revendications précédentes, **caractérisée en ce que** le système (VE) de production d'image en volume est conçu, pour la détermination d'une valeur (VW) d'un voxel (V), pour remplacer une valeur (PW1) d'un premier pixel (P1), dont le rayon (PS1) de projection est en intersection dans le voxel (V) avec le rayon (PS2) de projection d'un deuxième pixel (P2), par la deuxième valeur (PW2) du deuxième pixel (P2), si la valeur (VW1) de confiance de la première valeur (PW1) de pixel est inférieure à une première valeur de seuil, et/ou **en ce que** le système (VE) de production d'image en volume est préparé pour remplacer une valeur (PW2) d'un deuxième pixel (P2), dont le rayon (PS2) de projection est en intersection dans le voxel (V) avec le rayon (PS1) de projection d'un premier pixel (P1), par la première valeur (PW1) du premier pixel (P1), si la valeur (VW2) de confiance de la deuxième valeur (PW2) de pixel est inférieure à la première valeur de seuil.

4. Installation (TA) de tomographie suivant l'une des revendications précédentes, **caractérisée en ce que** le système (VE) de production d'image en volume est conçu, pour la détermination d'une valeur (VW) d'un voxel (V), en tenant compte de valeurs (PW1, PW2) de premiers (P1) et de deuxièmes (P2) pixels, dont les rayons (PS1, P2) de projection sont en intersection dans le voxel (V), pour remplacer la valeur (PW1) du premier (P1) par une interpolation dans le temps et/ou une interpolation entre des valeurs (PW1') de pixels (P1'), qui sont voisins dans l'espace du premier pixel (P1), si la valeur (VW1) de confiance de la première valeur (PW1) de pixel est inférieure à une deuxième valeur (SW2) de seuil et/ou pour remplacer la valeur (PW2) du deuxième pixel (P2) par une interpolation dans le temps et/ou par une interpolation entre des valeurs (PW2') de pixels (P2'), qui sont voisins dans l'espace du deuxième pixel (P2), si la valeur (VW2) de confiance de la deuxième valeur (PW2) de pixel est inférieure à la deuxième valeur (SW2) de seuil.

5. Installation (TA) de tomographie suivant l'une des revendications précédentes, **caractérisée en ce que** le système (VE) de production d'image en volume est conçu pour obtenir une première image (M1) de masque en trois dimensions à partir des jeux (PB1, PB2) de données d'image de projection du tour ou du tour partiel, en synchronisme et décalés en angle de rotation des deux paires (QD1, QD2) source de rayonnement-détecteur, en retirant des jeux (PB1, PB2) de données d'image de projection ou de données d'image en volume, qui en sont reconstruites, des données, qui se modifient pendant le tour ou le tour partiel des deux paires (QD1, QD2) de source de rayonnement-détecteur.

6. Installation (TA) de tomographie suivant l'une des revendications précédentes, **caractérisée en ce que** le système (V1) de production d'image en volume est conçu pour obtenir la succession (AV) des images (VB) en volume du système (GS) de vaisseau, en tenant compte d'une deuxième image (M2) de masque en trois dimensions, d'une course (ML) de masque et/ou pour l'obtenir, en tenant compte d'une troisième image (M3) de masque en trois dimensions, qui est rapportée à une source (ED) de données extérieures.

7. Installation (TA) de tomographie suivant la revendication 5 ou 6, **caractérisée en ce que** le système (VE) de production d'image en volume est conçu pour utiliser la première (M1) et/ou la deuxième (M2) et/ou la troisième (M3) images de masque en trois dimensions du système (GS) de vaisseau, comme image de démarcation pour la succession (AV) des images (VB) en volume.

8. Installation (TA) de tomographie suivant l'une des revendications précédentes, **caractérisée en ce que** le deuxième angle (PW2) de projection diffère du premier angle (PW1) de projection de 90°.

9. Procédé (100) de production d'une succession (AV) d'images (VB) en volume d'un système (GS) de vaisseau, le procédé (100) comprenant ce qui suit :
- détection (110), à partir d'un premier angle (W1) de projection, d'une succession (A1) de premiers jeux (PB1) de données d'image de projection ayant des premières valeurs (PW1) de pixel ;
- détection (120), à partir d'un deuxième angle (W2) de projection, d'une succession (A2) de deuxièmes jeux (PB2) de données d'image de projection ayant des deuxièmes valeurs (PW2) de pixel, le deuxième angle (W2) de projection étant différent du premier angle (W1) de projection, dans lequel
- on détecte les successions (A1, A2) des premiers (PB1) et deuxièmes (PB2) jeux de données d'image de projection pendant un tour ou un tour partiel, en synchronisme et décalés en angle de rotation, des deux paires (QD1, QD2) source de rayonnement-détecteur, dans lequel, pour les deux paires (QD1, QD2) source de rayonnement-détecteur, le détecteur (D1, D2) tourne dans un plan (RE1, RE2) de rotation, qui est à distance du plan (RE1, RE2) de rotation dans lequel tourne la source (Q1, Q2) de rayonnement associée au détecteur (D1, D2), dans lequel un rayon central de la source (Q1, Q2) de rayonnement est dirigé sur le détecteur (D1, D2) respectif, qui lui est associé, de la paire (QD1, QD2) source de rayonnement-détecteur respectif ;
- production (130) de la succession (AV) d'images (VB) en volume, en tenant compte de premières valeurs (VW1) de confiance, comme poids des premières valeurs (PW1) de pixel et/ou en tenant compte des deuxièmes valeurs (VW2) de confiance, comme poids des deuxièmes valeurs (PW2) de pixel, la valeur (VW1, VW2) de confiance d'une valeur (PW1, PW2) de pixel dépendant d'une longueur (L) de parcours, spécifique au pixel, qu'un rayon (PS1, PS2) de projection parcourt dans des parties (Gi) du système (GS) de vaisseau, sur un chemin allant de la première (Q1) ou de la deuxième (Q2) source de rayonnement à un élément (S) de capteur, spécifique au pixel, du premier (D1) ou du deuxième (D2) détecteur associé et la valeur (VW1, VW2) de confiance étant d'autant plus grande que le rayon (PS1, PS2) de projection a parcouru moins de parties (Gi) du système (GS) de vaisseau.
